# EUROPEAN PATENT APPLICATION

(11) **EP 1 961 434 A2**
(43) Date of publication of application: **27.08.2008**
(21) Application number: 08151553.8
(22) Date of filing: 18.02.2008
(51) Int. Cl.: A61L 31/10, A61L 31/14, A61L 31/16, A61L 33/00

(54) **A Coating for a Medical Device Having an Anti-Thrombotic Conjugate**

(30) Priority: 21.02.2007 US 677190
(71) Applicant: Cordis Corporation, Miami Lakes, FL 33014 (US)
(72) Inventor: Zhao, Jonathan Z., Belle Mead, NJ NJ 08502 (US); Falotico, Robert, Belle Mead, NJ NJ 08502 (US)
(74) Representative: Belcher, Simon James

(57) **Abstract**

A conjugate between an anti-thrombotic agent and a bioabsorbable polymer is provided. In addition, a method is provided for applying a coating comprising an anti-thrombotic agent and a bioabsorbable polymer conjugate to at least a portion of an implantable device to prevent or reduce the formation of thrombosis on the surface of the device. A first or sub-layer of the coating is prepared by mixing a polymeric material and a biologically active agent with a solvent, thereby forming a homogeneous solution. A second or outer layer comprises an anti-thrombotic heparin-bioabsorbable polymer conjugate. This coating may be applied over the inner drug-containing layers using, for example, a dip coating or spray coating process. After drying, the anti-thrombotic heparin bioabsorbable polymer conjugate remains in the outer layer of the coating, allowing agent from the inner layer to be eluted there through. In addition, the outmost layer prevents the formation of thrombosis, and also serves to modulate the release kinetics of the agent(s) contained within an inner layer(s) of the coating.

## Description

The present invention relates to a coating material for application to at least a portion of one surface of an article. In particular, this invention relates to anti-thrombotic and antirestenotic coating compositions having a multi-layered coating, wherein the first or inner layer is formed from a polymer and one or more biologically active agents, and a second or outer layer comprises an anti-thrombotic heparin-bioabsorbable polymer conjugate. This invention also relates to methods of making a heparin-bioabsorbable polymer conjugate and applying a coating material comprising such a anti-thrombotic heparin-bioabsorbable polymer conjugate over at lease a portion of the surface of an implantable medical device.

Atherogenic arterial narrowing (stenosis), and the gradual narrowing of a blood vessel following an angioplasty procedure or a stent implantation (restenosis) are tow commonly encountered vascular diseases. Stenosis refers to the narrowing or constriction of a vessel, which is usually due to the buildup of fat, cholesterol, and other substances over time. In severe cases, stenosis can completely clog a vessel. Thrombosis refers to the formation of blood clots on or near an implanted device in the blood vessel. The clot is usually formed by an aggregation of blood factors, primarily platelets and fibrin, with entrapment of cellular elements. Thrombosis, like stenosis, frequently causes vascular obstruction at the point of its formation. Both restenosis and thrombosis are two serious and potentially fatal conditions that need medical intervention.

One approach to clearing an artery that has been constricted or clogged due to stenosis is percutaneous transluminal coronary angioplasty (PTCA) or balloon coronary angioplasty. In this procedure, a balloon catheter is inserted and expanded in the constricted portion of the vessel for clearing the blockage. About one-third of patients who undergo PTCA suffer from restenosis, the renarrowing of the widened segment, within about six months of the procedure. Restenosed arteries may have to undergo another angioplasty.

Restenosis can be inhibited by a common procedure that consists of inserting a stent into the effected region of the artery instead of, or along with, angioplasty. A stent is a tube made of metal or plastic, which can have either solid walls or mesh walls. Most stents in use are metallic and are either self-expanding or balloon-expandable. The decision to undergo a stent insertion procedure depends on certain features of the arterial stenosis. These include the size of the artery and the location of the stenosis. The function of the stent is to buttress the artery that has recently been widened using angioplasty, or, if no angioplasty was used, the stent is used to prevent elastic recoil of the artery. Stents are typically implanted via a catheter. In the case of a balloon-expandable stent, the stent is collapsed to a small diameter and slid over a balloon catheter. The catheter is then maneuvered through the patient's vasculature to the site of the lesion or the area that was recently widened. Once in position, the stent is expanded and locked in place. The stent stays in the artery permanently, holds it open, improves blood flow through the artery, and relieves symptoms (usually chest pain).

Stents are not completely effective in preventing restenosis at the implant site. Restenosis can occur over the length of the stent and/or past the ends of the stent. Physicians have recently employed new types of stents that are coated with a thin polymer film loaded with a drug that inhibits smooth cell proliferation. The coating is applied to the stent prior to insertion into the artery using methods well known in the art, such as a solvent evaporation technique. The solvent evaporation technique entails mixing the polymer and drug in a solvent. The solution comprising polymer, drug, and solvent can then be applied to the surface of the stent by either dipping or spraying. The stent is then subjected to a drying process, during which the solvent is evaporated, and the polymeric material, with the drug dispersed therein, forms a thin film layer on the stent.

The release mechanism of the drug from the polymeric materials depends on the nature of the polymeric material and the drug to be incorporated. The drug diffuses through the polymer to the polymer-fluid interface and then into the fluid. Release can also occur through degradation of the polymeric material. The degradation of the polymeric material may occur through hydrolysis or an enzymatic digestion process, leading to the release of the incorporated drug into the surrounding tissue.

An important consideration in using coated stents is the release rate of the drug from the coating. It is desirable that an effective therapeutic amount of the drug be released from the stent for a reasonably long period of time to cover the duration of the biological processes following and an angioplasty procedure or the implantation of a stent. Burst release, a high release rate immediately following implantation, is undesirable and a persistent problem. While typically not harmful to the patient, a burst release "wastes" the limited supply of the drug by releasing several times the effective amount required and shortens the duration of the release period. Several techniques have been developed in an attempt to reduce burst release. For example, US-6,258,121 (Yang et al.) discloses a method of altering the release rate by blending two polymers with differing release rates and incorporating them into a single layer.

Another potential problem associated with the implantation of a drug eluting stent is thrombosis that may occur at different times following the implantation of a stent. A thrombus formation on the surface of a stent is frequently lethal, leading to a high mortality rate of between 20 to 40% in the patients suffering from a thrombosis in a vessel.

One way to address the formation of stent thrombosis is through the use of a potent anticoagulant such as a heparin. Heparin is a substance that is well known for its anticoagulation ability. It is known in the art to apply a thin polymer coating loaded with heparin onto the surface of a stent using the solvent evaporation technique. For example, US-5,837,313 (Ding et al.) describes a method of preparing a heparin coating composition. Unfortunately heparin because of its hydrophilic nature elutes out of the polymer matrix quickly without staying on the surface of the implant where the thrombosis occurs. The leaching of a heparin molecule and the infiltration of water into the polymer matrix where an anti-restenotic agent is contained may cause a rapid elution of the drug from the polymer matrix and consequently a less than desirable efficacy of the drug. The stability of the drug may also be adversely affected by the presence of water.

Therapeutic or biologically active agents, such as those used to prevent thrombosis, are included within a coating whereby after implantation of the device, the therapeutic agent will be eluted from the coating to the surrounding tissue of the body. Thus, the coating must allow the pharmaceutical or therapeutic agents to permeate there through. It is also desirable that the coating functions as a physical barrier, a chemical barrier, or a combination thereof to control the elution of the pharmaceutical or therapeutic agents from the underlying basecoat. This is accomplished by controlling the access of water and other fluids to the therapeutic agent. Absent regulation of fluid flow, the agent will elute more rapidly than desired. For example, if it is desired to have the agent released within a month, rapid hydration may lead to the agent being released within days.

Although effective in reducing restenosis, some of the components of the coatings utilized for a DES may increase the risk of thrombosis. Drug eluting stents are typically not associated with an increase of acute and subacute thrombosis (SAT), or a medium term thrombosis (30 days after stent implantation) following a stent implantation. Long term clinical follow up studies, however, suggest that these devices may be involved with increased incident rates of very long term thrombosis (LST). Although the increase of LST has been found to be less than 1%, a high mortality rate is usually associated with LST. One way to prevent this is to include a coating of an anti-coagulant, such as heparin, on the device.
Few devices can deliver an agent to prevent restenosis while also ensuring that the coatings that the agent is embedded in will not contribute to thrombosis. An obvious solution is to combine the agent with an anti-coagulant within a coating, however this fails due to the hydrophilic nature of anti-coagulants. For example, therapeutic agent is embedded in the matrix of a polymer coating by solvent processing. If an anti-coagulant is also embedded in the polymer matrix, it will attract water in an uncontrolled manner. This can happen during manufacturing or when the coated device is implanted and will adversely affect the stability or efficacy of the agent and/or interfere with the desired elution profile.

Nonetheless, several approaches have been proposed for combining anti-thrombotic and therapeutic agents within the coatings for an implantable medical device. US-5,525,348 (Whitbourne) discloses a method of complexing pharmaceutical agents (including heparin) with quaternary ammonium components or other ionic surfactants and bound with water insoluble polymers as an antithrombotic coating composition. This method suffers from the possibility of introducing naturally derived polymer such as cellulose, or a derivative thereof, which is heterogeneous in nature and may cause unwanted inflammatory reactions at the implantation site. These ionic complexes between an antithrombotic agent such as heparin and an oppositely charged carrier polymer may also negatively affect the coating integration, and if additional pharmaceutical agents are present, may affect the shelf stability and release kinetics of these pharmaceutical agents.

A slightly different approach is disclosed in US-6,702,850, US-6,245,753, and US-7,129,224 (Byun) wherein antithrombotic agents, such as heparin, are covalently conjugated to a non-absorbable polymer, such as a polyarylic acid, before use in a coating formulation. The overall hydrophobicity of these conjugates is further adjusted by addition of a hydrophobic agent such as octadecylamine, which is an amine with a long hydrocarbon chain. This approach has several potential disadvantages such as the known toxicity of polyacrylic acid after heparin is metabolized in vivo. The addition of a hydrophobic amine also raises the concern of tissue compatibility and reproduction of the substitution reactions of each step. Moreover, the remaining components of the coating are not biodegradable.

Another antithrombotic coating approach is disclosed in US-6,559,132 (Holmer), US-6,461,665 (Scholander), and US-6,767,405 (Eketrop) whereby a carrier molecule such as chitosan is conjugated to an activated metal surface of a medical device. Thereafter, heparin is covalently conjugated to an intermediate molecule. This process may be repeated several times until a desired antithrombotic layer is achieved. Alternatively, this coating can be achieved in a batch process mode. This approach, however, is not readily applicable to a medical device that is coated with a polymer coating that contains pharmaceutical agent/s. Some of these successful anti-restenotic agents such as sirolimus may be damaged during these conjugating processes, especially these processes where aqueous processes are involved.

PCT application WO-A-2005/097223 (Stucke et al), discloses a method wherein a mixture of heparin conjugated with photoactive crosslinkers with dissolved or dispersed with other durable polymers such as Poly(butyl methacrylate) and poly(vinyl pyrrolidone) in a same coating solution and crosslinked with UV light in the solution or after the coating is applied. The potential disadvantage of this approach is that the incorporated drug/s may be adversely affected by the high energy UV light during crosslinking process, or worse, the drug/s may be crosslinked to the matrix polymers if they possess functional groups that may be activated by the UV energy.

Another general approach as disclosed in US-A-2005/0191333, US-A-2006/0204533 and WO-A-2006/099514 (all by Hsu, Li-Chien, et al.), uses a low molecular weight complex of heparin and a counter ion (stearylkonium heparin), or a high molecular weight polyelectrolyte complex , such as dextran, pectin to form a complex form of an antithrombotic entity. These antithrombotic complexes are further dispersed in a polymer matrix which may further comprise a drug. Such approaches create a heterogeneous matrix of a drug and a hydrophilic species of heparin wherein the hydrophilic species attract water before and after the implantation to adverse the stability and release kinetics of the drug. In addition, the desired antithrombotic functions of heparin and similar agent should be preferably located on the surface, not being eluted away from the surface of a coated medical device.

Thus, there remains a need for a coating material that can satisfy the stringent requirements, as described above, for applying on at least one surface of a medical device and can be prepared through a process that is compatible with the sensitive pharmaceutical or therapeutic agents impregnated in the coatings. This helps to fill a need for a coating that treats both restenosis and prevents thrombosis when applied to the outer surface of a drug eluting stent.

A conjugate between a heparin and a bioabsorbable polymer with a free carboxyl end group is provided. In addition, a method is provided for applying a coating comprising a heparin bioabsorbable polymer conjugate to at least a portion of an implantable device to prevent or reduce the formation of thrombosis on the surface of the device. The outmost layer of the coating comprises the conjugate of the present invention, which prevents the formation of thrombosis, and also serves to modulate the release kinetics of the agent(s) contained within an inner layer(s) of the coating.

A first or sub-layer of the coating is prepared by mixing a polymeric material and a biologically active agent with a solvent, thereby forming a homogeneous solution. The polymeric material can be selected from a wide range of synthetic materials, but in one exemplary embodiment, a poly(lactide-to-glycolide) (PLGA) is used. The biologically active agent is selected depending on the desired therapeutic results. For example, an antiproliferative drug such as paclitaxel, an immunosuppressant, such as a rapamycin, and/or anti-inflammatory drug, such as dexamethasone, may be included in the inner layer. Once prepared, the solution can be applied to the device through a dipping or spraying process. During drying, the solvent evaporates, and a thin layer of the polymeric material loaded with the biologically active agent is left coated over the stent. It should be noted that the current invention is not limited to just one inner layer or biologically active agent per layer. It is within the scope of this invention to add one or more distinct biologically active agents to each layer and/or have more than one inner layer loaded with a biologically active agent.

The second or outer layer comprises an anti-thrombotic heparin-bioabsorbable polymer conjugate. This coating may be applied over the inner drug-containing layers using, for example, a dip coating or spray coating process. In one exemplary embodiment of the present invention, the outer layer comprising an anti-thrombotic heparin-bioabsorbable polymer conjugate may be dissolved in a mixed solvent system comprising ethyl acetate (EA) and isopropanol (IPA). The solution is then sprayed onto the surface of the device that has already been coated with the agent-containing layer as described above. After drying, the antithrombotic heparin bioabsorbable polymer conjugate remains in the outer layer of the coating, allowing agent from the inner layer to be eluted there through.

The coated device is inserted into an afflicted area of a body, for example, a vessel like the coronary artery, using an appropriate procedure that depends on the properties of the device. Once in place, the device will hold the vessel open. The biologically active agent will be released from the first layer, thereby providing the desired therapeutic result, such as inhibiting smooth cell proliferation. The anti-thrombotic heparin-bioabsorbable polymer conjugate in the outmost layer becomes partially hydrated and prevents blood coagulation on and around the device, thus inhibiting thrombosis and subacute device thrombosis. In addition, the anti-thrombotic heparin-bioabsorbable polymer conjugate in the outmost layer may additionally reduce or prevent the burst release of the biologically active agent from the inner drug containing layer, thereby allowing the release to occur over a relatively extended period of time.

Embodiments of the present invention will now be described by way of example with reference to the accompanying drawings, in which:
Figure 1 is a schematic representation of ring opening polymerization of mixture of lactide and glycolide dimers with water as the initiator to form a carboxyl ended bioabsorbable polymer (PLGA).
Figure 2 is another schematic of a conjugation eaction of a carboxyl ended polymer and a heparin molecule.
Figure 3 is a schematic of a coating configuration applied to the surface of a medical device with the conjugate of the present invention being present in an outer layer.

One or more layers of polymeric compositions are applied to a medical device to provide a coating thereto. The polymeric compositions perform differing functions. For example, one layer may comprise a base coat that allows additional layers to adhere thereto. An additional layer(s) can carry bioactive agents within their polymer matrices. Alternatively, a single coat may be applied wherein the polymeric composition is such that the coat performs multiple functions, such as allowing the coating to adhere to the device and housing an agent that prevents thrombosis. Other functions include housing an agent to prevent restenosis. Often, however, the chemical requirement of each agent limits the number of agents a coating may carry. For example, an antithrombotic agent tends to be hydrophilic while an antiproliferative agent tends to be comparatively hydrophobic. Hence, it is desired to entrap a hydrophobic agent within the matrix of a polymer coating to limit its exposure to water and control its elution from the matrix. The present invention maintains two agents having differing properties in close proximity by providing a conjugate between a heparin and a bioabsorbable polymer with a free carboxyl end group. When coated onto a medical device the conjugate ensures that the anti-thrombotic agent is substantially oriented away from any hydrophobic agents that may be contained within the polymer matrix.

The following definitions are provided for ease of understanding the present invention and should not be construed as limiting the description of then invention in any way.

As used herein, "stent" means a generally tubular structure constructed from any biocompatible material that is inserted into a conduit to keep the lumen open and prevent closure due to a stricture or external compression.

As used herein, "biologically active agent" means a drug or other substance that has therapeutic value to a living organism including without limitation antithrombotics, anticancer agents, anticoagulants, antiplatelet agents, thrombolytics, antiproliferatives, antiinflammatories, agents that inhibit restenosis, smooth muscle cell inhibitors, antibiotics, and the like, and/or mixtures thereof and/or any substance that may assist another substance in performing the function of providing therapeutic value to a living organism.

Exemplary anticancer drugs include acivicin, aclarubicin, acodazole, acronycine, adozelesin, alanosine, aldesleukin, allopurinol sodium, altretamine, aminoglutethimide, amonafide, ampligen, amsacrine, androgens, anguidine, aphidicolin glycinate, asaley, asparaginase, 5-azacitidine, azathioprine, Bacillus calmette-guerin (BCG), Baker's Antifol (soluble), beta-2'-deoxythioguanosine, bisantrene hcl, bleomycin sulfate, busulfan, buthionine sulfoximine, ceracemide, carbetimer, carboplatin, carmustine, chlorambucil, chloroquinoxaline-sulfonamide, chlorozotocin, chromomycin A3, cisplatin, cladribine, corticosteroids, Corynebacterium parvum, CPT-11, crisnatol, cyclocytidine, cyclophosphamide, cytarabine, cytembena, dabis maleate, dacarbazine, dactinomycin, daunorubicin HC1, deazauridine, dexrazoxane, dianhydrogalactitol, diaziquone, dibromodulcitol, didemnin B, diethyldithiocarbamate, diglycoaldehyde, dihydro-5-azacytidine, doxorubicin, echinomycin, edatrexate, edelfosine, eflomithine, Elliott's solution, elsamitrucin, epirubicin, esorubicin, estramustine phosphate, estrogens, etanidazole, ethiofos, etoposide, fadrazole, fazarabine, fenretinide, filgrastim, finasteride, flavone acetic acid, floxuridine, fludarabine phosphate, 5-fluorouracil, Fluosol®, flutamide, gallium nitrate, gemcitabine, goserelin acetate, hepsulfam, hexamethylene bisacetamide, homoharringtonine, hydrazine sulfate, 4-hydroxyandrostenedione, hydrozyurea, idarubicin HC1, ifosfamide, interferon alfa, interferon beta, interferon gamma, interleukin-1 alpha and beta, interleukin-3, interleukin-4, interleukin-6, 4-ipomeanol, iproplatin, isotretinoin, leucovorin calcium, leuprolide acetate, levamisole, liposomal daunorubicin, liposome encapsulated doxorubicin, lomustine, lonidamine, maytansine, mechlorethamine hydrochloride, melphalan, menogaril, merbarone, 6-mercaptopurine, mesna, methanol extraction residue of Bacillus calmette-guerin, methotrexate, N-methylformamide, mifepristone, mitoguazone, mitomycin-C, mitotane, mitoxantrone hydrochloride, monocyte/macrophage colony-stimulating factor, nabilone, nafoxidine, neocarzinostatin, octreotide acetate, ormaplatin, oxaliplatin, paclitaxel, pala, pentostatin, piperazinedione, pipobroman, pirarubicin, piritrexim, piroxantrone hydrochloride, PIXY-321, plicamycin, porfimer sodium, prednimustine, procarbazine, progestins, pyrazofurin, razoxane, sargramostim, semustine, spirogermanium, spiromustine, streptonigrin, streptozocin, sulofenur, suramin sodium, tamoxifen, taxotere, tegafur, teniposide, terephthalamidine, teroxirone, thioguanine, thiotepa, thymidine injection, tiazofurin, topotecan, toremifene, tretinoin, trifluoperazine hydrochloride, trifluridine, trimetrexate, tumor necrosis factor, uracil mustard, vinblastine sulfate, vincristine sulfate, vindesine, vinorelbine, vinzolidine, Yoshi 864, zorubicin, and mixtures thereof.

Exemplary anti-inflammatory drugs include classic non-steroidal anti-inflammatory drugs (NSAIDS), such as aspirin, diclofenac, indomethacin, sulindac, ketoprofen, flurbiprofen, ibuprofen, naproxen, piroxicam, tenoxicam, tolmetin, ketorolac, oxaprosin, mefenamic acid, fenoprofen, nambumetone (relafen), acetaminophen (Tylenol®), and mixtures thereof; COX-2 inhibitors, such as nimesulide, NS-398, flosulid, L-745337, celecoxib, rofecoxib, SC-57666, DuP-697, parecoxib sodium, JTE-522, valdecoxib, SC-58125, etoricoxib, RS-57067, L-748780, L-761066, APHS, etodolac, meloxicam, S-2474, and mixtures thereof; glucocorticoids, such as hydrocortisone, cortisone, prednisone, prednisolone, methylprednisolone, meprednisone, triamcinolone, paramethasone, fluprednisolone, betamethasone, dexamethasone, fludrocortisone, desoxycorticosterone, and mixtures thereof; and mixtures thereof.

As used herein, "polymer" means a macromolecule made of repeating monomer units or co-monomer units.

As used herein, "macromolecule" means synthetic macromolecules, proteins, biopolymers and other molecules with a molecular weight typically greater than 1000.

As used herein, "effective amount" means an amount of pharmacologically active agent that is nontoxic but sufficient to provide the desired local or systemic effect and performance at a reasonable benefit/risk ratio attending any medical treatment.

In an exemplary embodiment of the present invention, a first or inner layer of a coating comprises a polymeric film loaded with a biologically active agent that prevents smooth cell proliferation and migration, such as a rapamycin. One manner in which the agent is placed within the matrix of the polymer involves using a solvent or mixture of solvents whereby the agent and polymer are dissolved therein. As the mixture dries, the solvent is removed leaving the agent entrapped within the matrix of the polymer. Exemplary polymers that can be used for making the inner/ first polymeric layer include polyurethanes, polyethylene terephthalate (PET), PLLA-poly-glycolic acid (PGA) copolymer (PLGA), polycaprolactone (PCL) poly-(hydroxybutyrate-co-hydroxyvalerate) copolymer (PHBV), poly(vinylpyrrolidone) (PVP), polytetrafluoroethylene (PTFE, Teflon®), poly(2-hydroxyethylmethacrylate) (poly-HEMA), poly(etherurethane urea), silicones, acrylics, epoxides, polyesters, urethanes, polyphosphazene polymers, fluoropolymers, polyamides, polyolefins, and mixtures thereof. Exemplary bioabsorbable polymers that can be used for making the inner/ first polymeric film include polycaprolactone (PCL), poly-D, L-lactic acid (DL-PLA), poly-L-lactic acid (L-PLA), poly(hydroxybutyrate), polydioxanone, polyorthoester, polyanhydride, poly(glycolic acid), polyphosphoester, poly (amino acids), poly(trimethylene carbonate), poly(iminocarbonate), polyalkylene oxalates, polyphosphazenes, and aliphatic polycarbonates.

The second or outmost layer may comprise an anti-thrombotic heparin-bioabsorbable polymer conjugate with strong anticoagulation properties. The second layer of anti-thrombotic heparin-bioabsorbable polymer conjugate may additionally have the effect of preventing a burst release of the biologically active agent dispersed in the first or inner layer, resulting in a relatively longer release period of the biologically active agent. The first layer may contain more than one biologically active agent.

For purposes of illustrating the present invention, the coating(s) are applied to a medical device such as stents and/or stent-graft. It is also to be understood that any substrate, medical device, or part thereof having contact with organic fluid, or the like, may also be coated with the present invention. For example, other devices such as vena cava filters and anastomosis devices may be used with coatings having agents therein or the devices themselves may be fabricated with polymeric materials that have the drugs contained therein. Any of the stents or other medical devices described herein may be utilized for local or regional drug delivery. Balloon expandable stents may be utilized in any number of vessels or conduits, and are particularly well suited for use in coronary arteries. Self-expanding stents, on the other hand, are particularly well suited for use in vessels where crush recovery is a critical factor, for example, in the carotid artery.

In general, a metal stent, such as those manufactured from stainless steel, cobalt chromium alloys, but plastic or other appropriate materials may be used, however, the coating may also be applied to a polymeric stent. In one embodiment, the stent is a L605 cobalt chromium alloy. It is desirable, but not required, that the first and second coatings cover at least a portion of the entire stent surface. The application of the first layer is accomplished through a solvent evaporation process or some other known method. The solvent evaporation process entails combining the polymeric material and the biologically active agent with a solvent, such as tetrahydrofuran (THF), which are then mixed by stirring to form a mixture. An illustrative polymeric material of the first layer comprises polyurethane and an illustrative biologically active agent comprises a rapamycin. The mixture is then applied to the surface of the stent by either: (1) spraying the solution onto the stent; or (2) dipping the stent into the solution. After the mixture has been applied, the stent is subjected to a drying process, during which, the solvent evaporates and the polymeric material and biologically active agent form a thin film on the stent. Alternatively, a plurality of biologically actives agent can be added to the first layer.

The second or outmost layer of the stent coating comprises an anti-thrombotic heparin-bioabsorbable polymer conjugate. The anti-thrombotic heparin-bioabsorbable polymer conjugate may be soluble in organic solvents or mixtures of organic solvents of varying polarity. The heparin may comprise an unfractionated heparin, fractionated heparin, a low molecular weight heparin, a desulfated heparin and heparins of various mammalian sources. Exemplary anti-thrombotic agents may include: Vitamin K antagonist such as Acenocoumarol, Clorindione, Dicumarol (Dicoumarol), Diphenadione, Ethyl biscoumacetate, Phenprocoumon, Phenindione, Tioclomarol, Warfarin; Heparin group anti-platelet aggregation inhibitors such as Antithrombin III, Bemiparin, Dalteparin, Danaparoid, Enoxaparin, Heparin, Nadroparin, Parnaparin, Reviparin, Sulodexide, Tinzaparin; other platelet aggregation inhibitors such as Abciximab, Acetylsalicylic acid (Aspirin), Aloxiprin, Beraprost, Ditazole, Carbasalate calcium, Cloricromen, Clopidogrel, Dipyridamole, Eptifibatide, Indobufen, Iloprost, Picotamide, Prasugrel, Prostacyclin, Ticlopidine, Tirofiban, Treprostinil, Triflusal; enzymatic anticoagulants such as Alteplase, Ancrod, Anistreplase, Brinase, Drotrecogin alfa, Fibrinolysin, Protein C, Reteplase, Saruplase, Streptokinase, Tenecteplase, Urokinase; direct thrombin inhibitors such as Argatroban, Bivalirudin, Dabigatran, Desirudin, Hirudin, Lepirudin, Melagatran, Ximelagatran, and other antithrombotics such as Dabigatran, Defibrotide, Dermatan sulfate, Fondaparinux, Rivaroxaban.

In an exemplary embodiment, the anti-thrombotic heparin-bioabsorbable polymer conjugate is prepared as follows. First, a cyclic dimer of d,l-lactide, is polymerized at elevated temperature of about 140C, in the presence of a catalyst Stannous Octoate (Sn(OCt)₂ and a predetermined amount of water as the ring opening initiator. Ring opening polymerization results in an end product that contains a homopolymer of polyester. The molecular weight of each polymer is determined by the ratio between the cyclic dimer and the initiator. The higher the ratio between the cyclic dimer to the initiator, the higher the molecular weight of the polymer. The initiator used in ring opening polymerization determines the end groups of the polymerized polyester. A mono-functional initiator such as ethanol will lead to a final polymer with only one hydroxyl group in the end. A di-functional initiator, such as ethylene glycol, will lead to a polymer with hydroxyl groups on both ends.

In one embodiment of the present invention an initiator, such as water, creates a carboxyl group at one end of the final polymer that may be further, and easily, employed in the subsequent conjugation reaction with a heparin molecule. The bioabsorbable polymer with a carboxyl end group synthesized in the fist step, may be activated by using N,N-dicyclohexylcarbodiimide hydrochloride (DDC) and N,N-hydroxysuccinimide (NHS) before the coupling reaction with the amine groups of a heparin molecule. Although any heparin molecule, a recombinant heparin, heparin derivatives or heparin analogues (having a preferred weight of 1,000-1,000,000 daltons) may be used in the coupling reaction to make the final anti-thrombotic heparin-bioabsorbable polymer conjugate, it is preferred to use a desulfated heparin to increase the coupling efficiency of the reaction.

Once the anti-thrombotic heparin-bioabsorbable polymer conjugate is prepared, the second layer comprising the anti-thrombotic heparin polymer conjugate may be applied directly over the first layer using the solvent evaporation method or other appropriate method. After the solvent is evaporate from the surface of an implantable medical device, a thin film of comprising anti-thrombotic heparin-bioabsorbable polymer conjugate is formed on the outmost surface of the device.

The following examples illustrate the creation of the conjugate in accordance with the principle of the present invention.

### I. Example 1

### Preparation Of A Bioabsorbable Polymer With Carboxyl End Groups

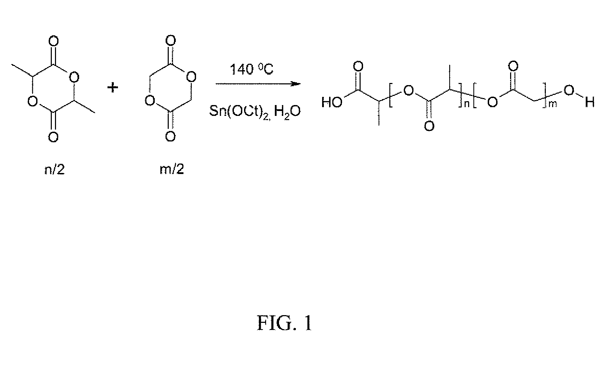

A pre-determined amount of d,l-lactide (from Purac USA) is transferred to a dried round bottom glass reactor equipped with a magnetic stir bar. A pre-determined amount of water and a toluene solution containing Stannous Octoate are added to the glass reactor. The glass reactor is then sealed with a stopper and cycled three times between an argon gas and vacuum to remove the air and oxygen inside the reactor. The sealed reactor is then gradually heated to 140C under vacuum and kept stirred with the magnetic stir bar. Upon completion of the reaction, the polymer is dissolved in methylene chloride and precipitated in ethanol and dried under vacuum and low heat. The process is schematically illustrated in Figure 1.

### II. Example 2

### Preparation Of Anti-Thrombotic Heparin-Bioabsorbable Polymer Conjugate

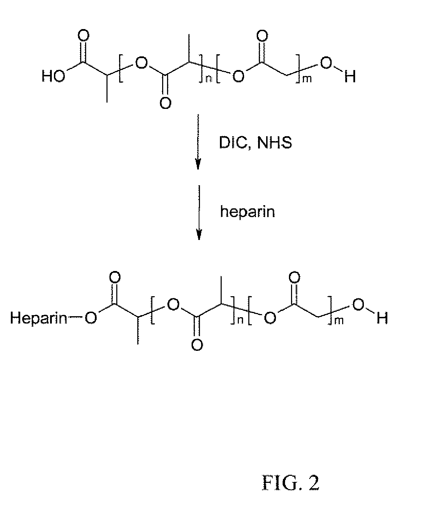

The bioabsorbable polymer made in example 1 is dissolved in dimethylformamide (DMF), followed by dissolution ofN-hydroxylsuccinimide (NHS) and dicyclohexylcarbodiimide (DCC). The mole ratio of PLA, NHS, and DCC is 1: 1. 6:1.6. The resulting solution is kept for 5 hours at room temperature under vacuum. The byproduct, dicyclohexylurea (DCU), and unreacted DCC and NHS are removed by filtration and extraction with water. The activated bioabsorbable polymer is then dissolved in DMF and reacted with heparin for 4 hours at room temperature. The final heparin PLGA conjugate is then precipitated and freeze dried. The process is schematically illustrated in Figure 2.

### III. Example 3

### Coating Of A Drug Eluting Stent With An Outmost Layer Comprising A Heparin Absorbable Polymer Conjugate

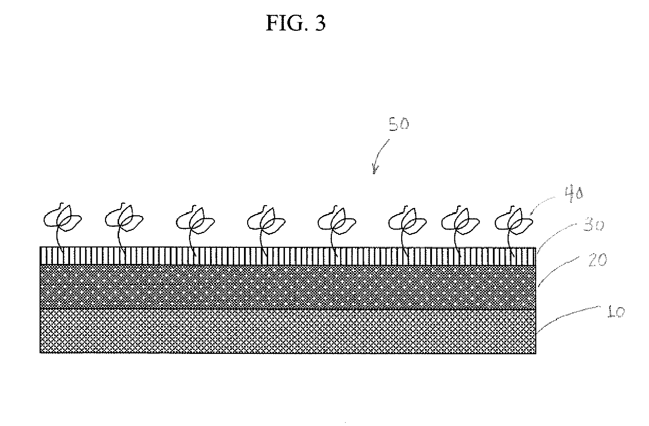

A coated medical device 50 in accordance with the present invention is schematically illustrated in Figure 3. The surface 10 of a cobalt chromium stent is spray coated with a drug containing polymeric solution 20, which may comprise for example, ethyl acetate (EA) containing PLGA and rapamycin. The weight ratio between PLGA and rapamycin is 2:1. After the drug-containing layer 20 is dried, a coating solution 30 containing a heparin absorbable polymer conjugate is spray coated onto the first drug-containing layer 20. After the coating solution 30 is dried, it will result in a thin film with the heparin 40 located substantially on the outmost surface.

## Claims

1. A conjugate material comprising an anti-thrombotic agent and a hydrophobic bioabsorbable polymer.

2. The conjugate material of claim 1, wherein the anti-thrombotic agent is heparin.

3. The conjugate material of claim 2 wherein the heparin is a low molecular weight heparin.

4. The conjugate material of claim 2 wherein the heparin is a de-sulfated heparin.

5. The conjugate material of claim 1 wherein bioabsorbable polymers comprise a copolymer.

6. The conjugate material of claim 5 wherein the copolymer is selected from a group consisting of poly(lactide-co-glycolide), poly(hydroxybutyrate-co-hydoxyvalerate), poly(glycolic acid-co-trimethylene carbonate), polyphosphoester urethane, and poly(ether-co-ester).

7. The conjugate material of claim 1 wherein the polymer is a homopolymer.

8. The conjugate material of claim 7 wherein the homopolymer is selected from a group consisting of polycaprolactone (PCL), poly-D, L-lactic acid (DL-PLA), poly-L-lactic acid (L-PLA), poly(hydroxybutyrate), poly(hydoxyvalerate), polydioxanone, polyorthoester, polyanhydride, poly(glycolic acid), polyphosphoester, poly (amino acids), poly(trimethylene carbonate), poly(iminocarbonate), polyalkylene oxalates, polyphosphazenes, and aliphatic polycarbonates.

9. The conjugate material of claim 1 wherein the bioabsorbable polymer comprises a polyester copolymer and the anti-thrombotic agent comprises a heparin molecule, the conjugate having the following structure: 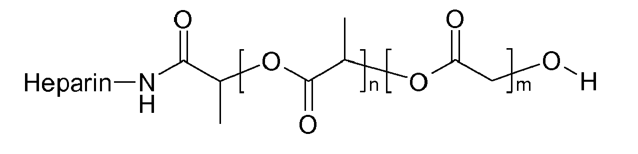 wherein n and m are independently an integer of 1 to 1000.

10. The conjugate material of claim 1 wherein the bioabsorbable polymer comprises a poly(lactide) and the anti-thrombotic agent comprises a heparin molecule, the conjugate having the following structure: 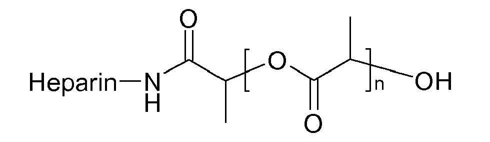 wherein n is an integer of 1 to 1000.

11. The conjugate of claim 1 wherein the bioabsorbable polymer comprises a terpolymer of 1,1-polylactide glycolide, glycolide, and caprolactone.

12. The conjugate of claim 1 wherein the terpolymer comprises a terpolymer of d, 1-polylactide glycolide, glycolide, and caprolactone.

13. A coating comprising:
a first bioabsorbable polymer applied to a surface being coated
an agent contained within the first bioabsorbable polymer; and
a conjugate of an anti-thrombotic molecule and a second, hydrophobic bioabsorbable polymer wherein the conjugate is applied to the top of the first bioabsorbable polymer

14. The coating of claim 15 wherein the anti-thrombotic molecule of the conjugate is substantially located distal from the first bioabsorbable polymer.

15. The coating of claim 15, wherein the anti-thrombotic molecule comprises heparin and the analogs and derivatives thereof.

16. The coating of claim 15, wherein the agent is an anti-restenotic agent selected from a rapamycin, paclitaxel, pimecrolimus, and the analogs and derivatives thereof.

17. The coating of claim 15 wherein the first and second bioabsorbable polymer comprise a copolymer.

18. The conjugate material of claim 19 wherein the copolymer is selected from a group consisting of poly(lactide-co-glycolide), poly(hydroxybutyrate-co-valerate), poly(glycolic acid-co-trimethylene carbonate), polyphosphoester urethane, and poly(ether-co-ester).

19. The coating of claim 15 wherein the first and second bioabsorbable polymer is a homopolymer.

20. The coating of claim 21 wherein the homopolymer is selected from a group consisting of polycaprolactone (PCL), poly-D, L-lactic acid (DL-PLA), poly-L-lactic acid (L-PLA), poly(hydroxybutyrate), polydioxanone, polyorthoester, polyanhydride, poly(glycolic acid), polyphosphoester, poly (amino acids), poly(trimethylene carbonate), poly(iminocarbonate), polyalkylene oxalates, polyphosphazenes, and aliphatic polycarbonates.

21. The coating of claim 15 wherein the first bioabsorbable polymer comprises a copolymer and the second bioabsorbable polymer comprises a homopolymer.

22. The coating of claim 15 wherein the first bioabsorbable polymer comprises a homopolymer and the second bioabsorbable polymer comprises a copolymer.

23. The coating of claim 15 wherein the conjugate comprises a polyester copolymer and the anti-thrombotic agent comprises a heparin molecule, the conjugate having the following structure: 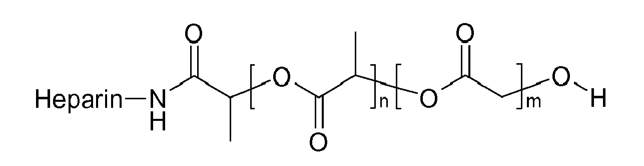 wherein n and m are independently an integer of 1 to 1000.

24. The coating of claim 15 wherein the coating is applied to an implantable medical device.

25. The coating of claim 26 wherein the medical device comprises a stent.

26. A method for forming a conjugate comprising the steps of:
using water as the initiator in a ring opening polymerization of at least one cyclic lactone molecule to create a bioabsorbable polymer with a carboxyl end group; and
conjugating the amine group of a heparin molecule to the carboxyl group of the bioabsorbable polymer.

27. The method of claim 28 wherein the at least one cyclic lactone molecule comprises a lactide.

28. The method of claim 28 wherein the at least one cyclic lactone molecule comprises a glycolide.

29. The method of claim 28 wherein the at least one cyclic lactone molecule comprises a caprolactone.

30. The method of claim 28 wherein a second lactone molecule is present.

31. The method of claim 28 wherein a coupling agent is utilized to facilitate the conjugation of the heparin and bioabsorbable polymer.
